# EUROPÄISCHE PATENTANMELDUNG

(11) **EP 3 181 100 A1**
(43) Veröffentlichungstag der Anmeldung: **21.06.2017**
(21) Anmeldenummer: 16202048.1
(22) Anmeldetag: 02.12.2016
(51) Int. Cl.: A61F 5/41, A61H 19/00

(54) **KUNSTPENIS**

(30) Priorität: 16.12.2015 DE 202015008591 U
(71) Anmelder: Kiefer, Andreas, 71032 Böblingen (DE)
(72) Erfinder: Kiefer, Andreas, 71032 Böblingen (DE)
(74) Vertreter: Puschmann Borchert Bardehle Patentanwälte Partnerschaft mbB

(57) **Zusammenfassung**

Kunstpenis (1) für eine männliche Person, der eine Vergrößerung des kleineren erigierten natürlichen Penis vortäuschen soll.

Der Kunstpenis (1) weist eine innere Aussparung (2) für die gedachte Aufnahme eines erigierten natürlichen Penis über eine Einführöffnung (3) auf.

Die Aussparung (2) umfasst einen eichelförmigähnlichen Aufnahme-Raum (2a) zur gedachten Aufnahme der Eichel des erigierten natürlichen Penis, und einen penisschaft-ähnlichen Aufnahme-Raum (2b) zur gedachten Aufnahme des an die Eichel anschließenden Penisschafts des erigierten natürlichen Penis.

Am Übergang vom Aufnahme-Raum (2a) zum Aufnahme-Raum (2b) ist ein Haltewulst (4) angeordnet, welcher an der Eichelböschung eines gedachten in den Kunst-Penis eingeführten erigierten natürlichen Penis anliegt. Der Kunstpenis (1) weist eine Befestigungs-Komponente (1 K) mit einer dehnbaren elastischen Durchstecköffnung für einen Teil des Hodensacks mit den Hoden auf, mit der der Kunstpenis (1) am menschlichen Körper lösbar befestigbar ist.

## Beschreibung

Die Erfindung bezieht sich auf einen Kunstpenis zur Anbringung auf einem erigierten kleineren natürlichen Penis einer männlichen Person.

Zur Vermeidung von Missverständnissen sei eingangs bemerkt, dass mit der Bezeichnung Kunstpenis ein künstlicher Penis gemeint ist.

Die äußeren Geschlechtsorgane des Mannes sind das Glied (Penis) und der Hodensack.

Der vorderste Teil des Penis wird Eichel genannt, welche bei nichterigiertem Penis nahezu vollständig von der zurückstreifbaren Vorhaut bedeckt wird. Bei Erektion streift sich die Vorhaut von allein hinter die Eichel zurück. Der hinter der Eichel liegende Teil des erigierten Penis sei Penisschaft genannt. Der obere Eichelrand fällt zum Penisschaft hin ab. Dieser Abfall sei hier mit Penis-Böschung bezeichnet.

Es ist Aufgabe der Erfindung, einen Kunstpenis für eine männliche Person vorzusehen, der eine Vergrößerung eines kleineren erigierten natürlichen Penis vortäuschen soll.

Diese Aufgabe der Erfindung wird durch die im kennzeichnenden Teil des Anspruches 1 genannten Merkmalen in Verbindung mit den Oberbegriffsmerkmalen gelöst.

Vorteilhafte Weiterbildungen der Erfindung sind in den Unteransprüchen gekennzeichnet.
Weitere Vorteile, Merkmale und Anwendungsmöglichkeiten der vorliegenden Erfindung ergeben sich aus der nachfolgenden Beschreibung in Verbindung mit den in den Zeichnungen dargestellten Ausführungsbeispielen.

In der Beschreibung, in den Ansprüchen und in den Zeichnungen werden die in der unten aufgeführten Liste der Bezugszeichen verwendeten Begriffe und zugeordneten Bezugszeichen verwendet. In der Zeichnung bedeutet:

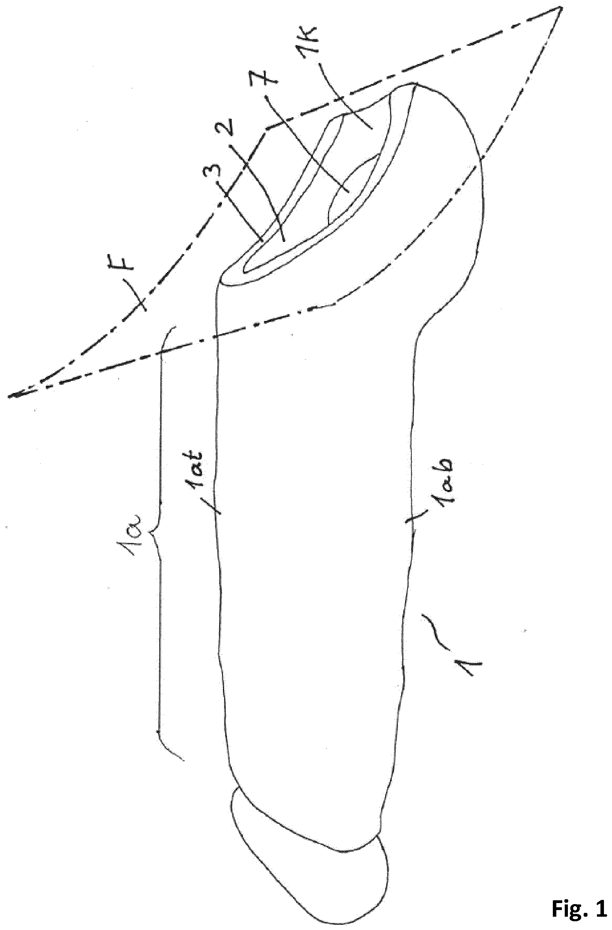

- Fig. 1: eine Seitenansicht des erfindungsgemäßen Kunstpenis in isometrischer Darstellung;
- Fig. 2: eine ausschnittsweise Schnittdarstellung des erfindungsgemäßen Kunstpenis mit SpermaKanal;
- Fig. 3: eine andere Seitenansicht des erfindungsgemäßen Kunstpenis in isometrischer Darstellung mit schräger Blickrichtung auf die gesamte Einführöffnung und die Befestigungs-Komponente;
- Fig. 4: eine Darstellung des erfindungsgemäßen Kunstpenis mit einer Entlüftungsrinne;
- Fig. 5A: eine Schnittdarstellung des erfindungsgemäßen Kunstpenis mit Sperma-Kanal gemäß der Schnittebene A-A in Fig.3;
- Fig. 5B: eine Schnittdarstellung des erfindungsgemäßen Kunstpenis mit Sperma-Kanal gemäß der Schnittebene B-B in Fig.3;
- Fig. 6A: eine Schnittdarstellung des erfindungsgemäßen Kunstpenis mit Entlüftungsrinne gemäß der Schnittebene C-C in Fig. 4;
- Fig. 6B: eine Schnittdarstellung des erfindungsgemäßen Kunstpenis mit Entlüftungsrinne gemäß der Schnittebene D-D in Fig. 4, und
- Fig. 7: eine Seitenansicht des Kunstpenis mit einer ausschnittsweisen Schnittdarstellung des Kunstpenis mit einer Entlüftungsrinne und einer Entlüftungsöffnung in der Wandung des Kunstpenis.

Der erfindungsgemäße Kunstpenis soll beim Geschlechtsverkehr die Vergrößerung eines erigierten natürlichen Penis vortäuschen. Der Kunstpenis 1 umfasst auch eine Befestigungs-Komponente 1 K, mit der dieser (1) am gedachten menschlichen Körper lösbar befestigbar ist, wie weiter unten noch ausgeführt wird.

Der Kunstpenis weist eine innere Aussparung 2 zur gedachten Aufnahme des erigierten natürlichen Penis über eine Einführöffnung 3 auf.

Diese Aussparung 2 umfasst im vorderen Teil des Kunstpenis 1 einen eichelförmigähnlichen Aufnahme-Raum 2a zur gedachten Aufnahme der Eichel des erigierten natürlichen Penis, und im Anschluss an den Aufnahme-Raum 2a in Richtung der Einführöffnung 3 einen penisschaft-ähnlichen Aufnahme-Raum 2b zur gedachten Aufnahme des an die Eichel anschließenden Penisschafts des erigierten natürlichen Penis.

Am Übergang vom Aufnahme-Raum 2a zum Aufnahme-Raum 2b im Innern der Aussparung 2 ist ein Haltewulst 4 angeordnet, welcher an der Eichelböschung eines gedachten in den Kunstpenis eingeführten erigierten natürlichen Penis anliegt und dadurch dem Kunstpenis in seinem vorderen Teil einen Halt gegen Abrutschen gibt. Der Kunstpenis 1 ist aus elastischem Material hergestellt, vorzugsweise aus Silikon.

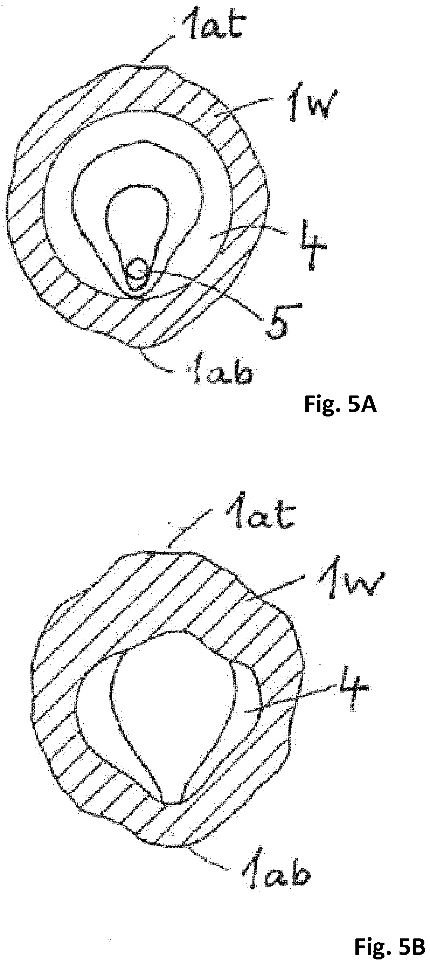

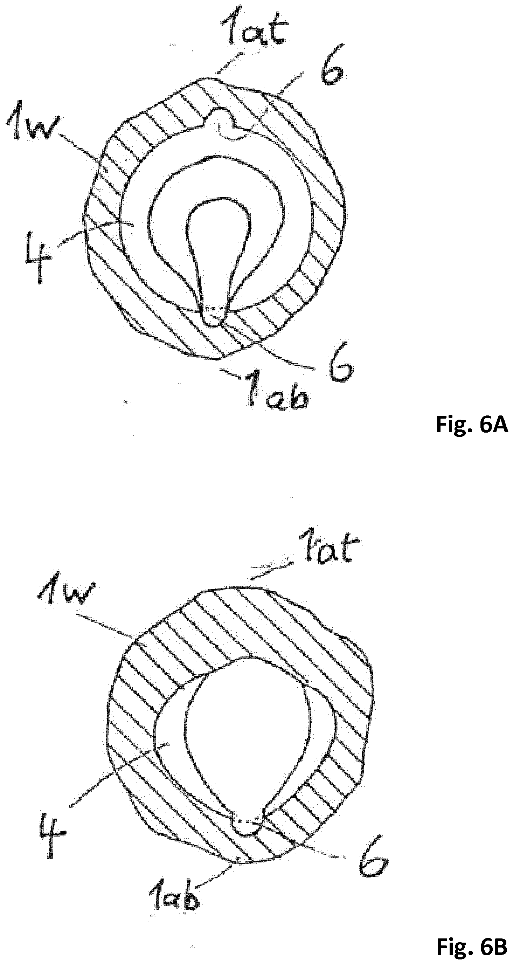

Der Haltewulst 4 ist im oberen Bereich 1 at des Kunstpenis 1 höher und schmaler als im unteren Bereich 1 ab des Kunstpenis 1 ausgebildet, siehe auch Fig. 5A, 5B, 6A, 6B.

Die Befestigungskomponente 1 K ist nahe der Einführöffnung 3 angeordnet. Die Einführöffnung (3) weist eine dehnbare elastische Durchstecköffnung 7 auf. Diese Durchstecköffnung (7) gestattet bei entsprechender Dehnung ein Hindurchstecken des Teiles des Hodensacks mit den Hoden, wodurch eine Befestigung des Kunstpenis 1 am menschlichen Körper bewirkt wird.

Diese Befestigung kann dadurch gelöst werden, dass bei entsprechender Dehnung der Durchsteck-Öffnung 7 der hindurchgesteckte Teil des Hodensacks mit den Hoden wieder durch diese Durchsteck-Öffnung 7 zurückgezogen wird.

Die Einführöffnung 3 weist eine ellipsenähnliche Form auf; sie liegt in einer gedachten gewölbten Fläche F, welche schräg und leicht gebogen zum hohlzylinderähnlichen Penisschaft 1 a des Kunstpenis 1 verläuft. Die Durchstecköffnung 7 ist in der Wandung des unteren Bereiches 1 a des Schaftes 1 a des Kunstpenis 1 angeordnet, auf den die Einführöffnung 3 projizierbar ist.

Der Bereich der Einführöffnung 3 des Kunstpenis ist vereinfacht gesehen mit dem schrägen Anschnitt eines liegenden Rohrendes vergleichbar, wobei der obere Rand der Öffnung den unteren Rand der Öffnung nicht überdeckt.

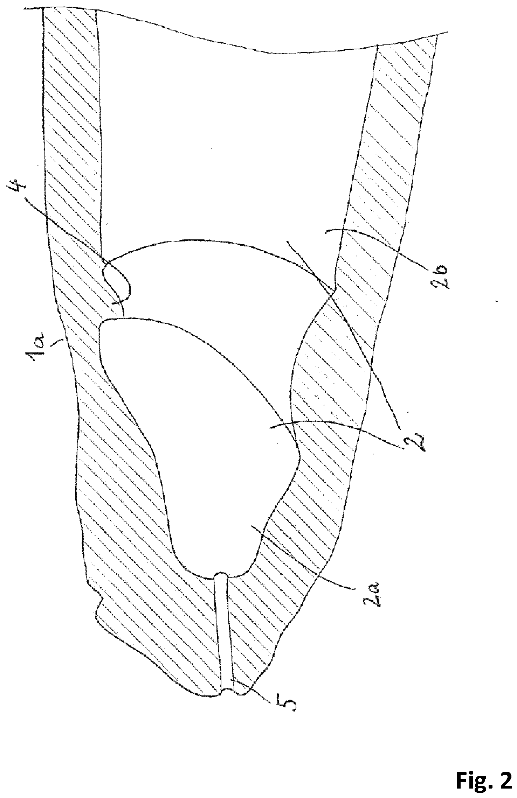

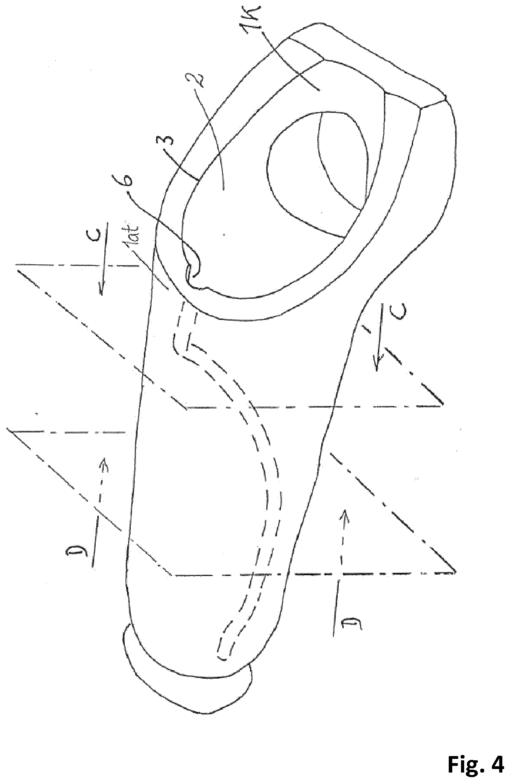

Aus Fig. 2 geht insbesondere die Aufteilung der inneren Aussparung 2 in den eichelförmigähnlichen Aufnahmeraum 2a und den penisschaft-ähnlichen Aufnahmeraum 2b sowie die Anordnung des Haltewulsts 4 hervor. Diese Struktur ist zwei Ausführungsformen der Erfindung gemeinsam. Die erste Ausführungsform weist wie in Fig. 2 gezeigt- zusätzlich einen Sperma-Kanal 5 auf, die zweite Ausführungsform gemäß Fig.4 weist zusätzlich eine Entlüftungs-Rinne 6 auf.

Aus Fig. 2 ist zu ersehen, dass der Sperma-Kanal 5 (er dient dem Abfluss des Ejakulats und der Entlüftung) vom vorderen Teil des eichelförmigähnlichen Aufnahmeraum 2a ausgeht und aus dem vorderen Teil der Eichel des Kunstpenis 1 austritt.

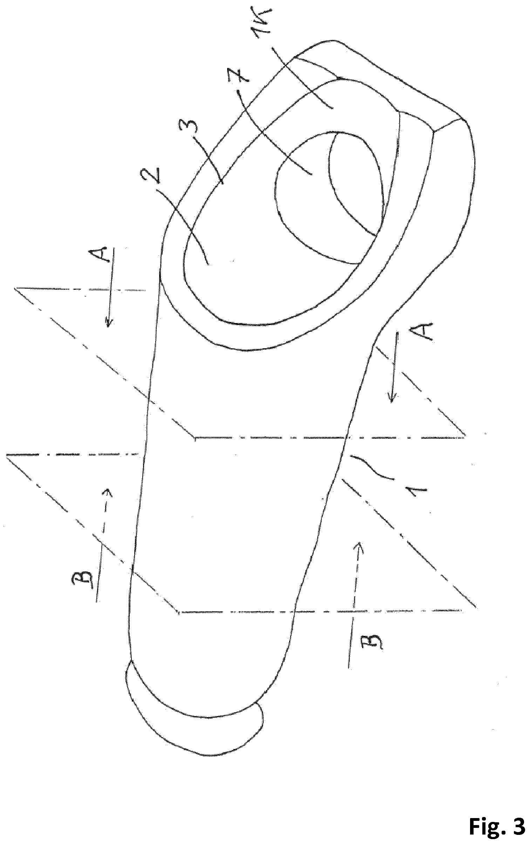

Fig. 4 zeigt eine isometrische Darstellung wie in Fig. 3 für eine Ausführungsform des Kunstpenis 1 mit einer Entlüftungsrinne 6.

Die Entlüftungsrinne 6 verläuft in der Wandung des Aufnahmeraumes 2a und des Aufnahmeraumes 2b bis zur Einführöffnung 3. Sie beginnt im vorderen Bereich des Aufnahmeraumes 2a und nimmt einen wendelförmigen Verlauf bis sie im oberen Bereich 1 at des Penisschaftes 1 a an der Einführöffnung 3 austritt. Die Entlüftungsrinne 6 ist zur Aussparung 2 hin offen. Sie kann ein entsprechend beliebiges Profil aufweisen.

Fig. 5A zeigt eine Schnittdarstellung des Kunstpenis 1 mit Sperma-Kanal 5 gemäß der Schnittebene A-A in Fig. 3. Die Schnittdarstellung erfolgt für die Blickrichtung A. In der Schnittdarstellung sind die Wandung des Kunstpenis 1 mit 1w, der Haltewulst mit 4 und der Sperma-Kanal mit 5 gekennzeichnet.

Fig. 5B zeigt eine Schnittdarstellung des Kunstpenis 1 mit Sperma-Kanal gemäß der Schnittebene B-B in Fig. 3. Die Schnittdarstellung erfolgt für die Blickrichtung B. In der Schnittdarstellung sind die Wandung des Kunstpenis 1 mit 1w und der Haltewulst mit 4 gekennzeichnet
Fig.6A zeigt eine Schnittdarstellung des Kunstpenis Entlüftungsrinne gemäß der Schnittebene C-C in Fig. 4. Die Schnittdarstellung erfolgt für die Blickrichtung C. In der Schnittdarstellung ist die Wandung des Kunstpenis 1 mit 1w, der Haltewulst mit 4 und die Entlüftungsrinne mit 6 gekennzeichnet.

Fig. 6B zeigt eine Schnittdarstellung des Kunstpenis 1 mit Entlüftungsrinne gemäß der Schnittebene D-D in Fig. 4. Die Schnittdarstellung erfolgt für die Blickrichtung D. In der Schnittdarstellung ist die Wandung des Kunstpenis 1 mit 1w, der Haltewulst mit 4 und die Entlüftungsrinne mit 6 gekennzeichnet.

In den Fig. 5A, 5B, 6A und 6B ist der obere Bereich des Kunstpenis mit 1 at, der untere Bereich mit 1 ab gekennzeichnet.

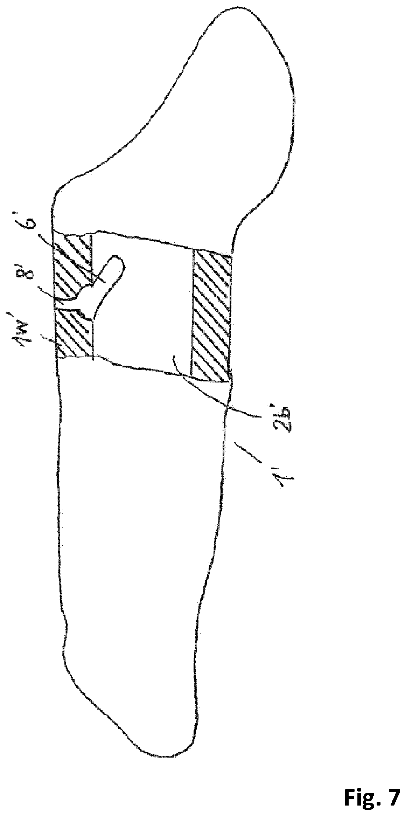

Fig. 7 zeigt eine Seitenansicht des Kunstpenis 1' mit einer ausschnittsweisen Schnittdarstellung des Kunstpenis mit einer Entlüftungsrinne 6' und einer Entlüftungsöffnung 8' in der Wandung 1w' des Kunstpenis 1'.

Die gedachte Schnittebene in Fig. 7 teilt den Kunstpenis 1' längs und verläuft durch den Endbereich der Entlüftungsrinne 6' und die Entlüftungsöffnung 8'.

Die Entlüftungsrinne 6'verläuft an der Innen-Wandung des eichelförmig-ähnlichen Aufnahmeraumes zur gedachten Aufnahme der Eichel des erigierten natürlichen Penis und an der Innen-Wandung 1w' des penisschaft-ähnlichen Aufnahmeraums 2b' zur gedachten Aufnahme des an die Eichel anschließenden Penisschaftes des erigierten natürlichen Penis.

Die Entlüftungsrinne beginnt im vorderen Bereich des Aufnahmeraumes für die Eichel und nimmt vorzugsweise einen wendelförmigähnlichen Verlauf bis in den Aufnahmebereich 2 b' hinein. Sie (6) endet an der Einführöffnung des Kunstpenis (wie in Fig. 4 dargestellt) oder sie (6') endet bereits vorher wie in Fig. 7 dargestellt.

Die Entlüftungsrinne 6' ist über mindestens eine Entlüftungsöffnung 8' in der Wandung 1w' des Kunstpenis 1' mit dem Bereich außerhalb des Kunstpenis 1' verbunden.

### Bezugszeichenliste

- 1, 1': Kunstpenis
- 1a: Kunstpenis-Schaft
- 1K: Befestigungs-Komponente
- 1at: oberer Bereich des Kunstpenis
- 1ab: unterer Bereich des Kunstpenis
- 1w, 1w*: Wandung
- 2: innere Aussparung
- 2a: eichelförmigähnlicher Aufnahmeraum
- 2b, 2b': penisschaftähnlicher Aufnahmeraum
- 3: Einführöffnung
- 4: Haltewulst
- 5: Sperma-Kanal
- 6: Entlüftungsrinne
- 7: Durchsteck-Öffnung
- F: gewölbte Fläche
- 8': Entlüftungsöffnung

## Patentansprüche

1. Kunstpenis, **dadurch gekennzeichnet, dass** der Kunstpenis (1) eine innere Aussparung (2) für die gedachte Aufnahme eines erigierten natürlichen Penis über eine Einführöffnung (3) aufweist,
dass die Aussparung (2) im vorderen Teil des Kunstpenis (1) einen eichelförmigähnlichen Aufnahme-Raum (2a) aufweist zur gedachten Aufnahme der Eichel des erigierten natürlichen Penis,
dass die Aussparung (2) im Anschluss an den Aufnahme-Raum (2a) in Richtung der Einführöffnung (3) einen penisschaft-ähnlichen Aufnahme-Raum (2b) aufweist zur gedachten Aufnahme des an die Eichel anschließenden Penisschafts des erigierten natürlichen Penis, dass am Übergang vom Aufnahme-Raum (2a) zum Aufnahme-Raum (2b) ein Haltewulst (4) angeordnet ist, welcher an der Eichelböschung eines gedachten in den Kunst-Penis (1) eingeführten erigierten natürlichen Penis anliegt,
dass der Kunstpenis (1) eine Befestigungs-Komponente (1 K) aufweist, mit der dieser (1) am gedachten menschlichen Körper lösbar befestigbar ist;
und dass der Kunstpenis (1) aus elastischem Material hergestellt ist.

2. Anordnung nach Anspruch 1, **dadurch gekennzeichnet, dass** die Befestigungs-Komponente (1 K) im Rand-Bereich der Einführöffnung (3) für den natürlichen Penis in den Kunstpenis (1) angeordnet ist und eine dehnbare elastische Durchstecköffnung (7) aufweist und
dass bei einer elastisch aufgeweiteten Durchsteck-Öffnung (7) der gedachte Teil des Hodensacks mit den Hoden die Durchsteck-Öffnung (7) passieren kann.

3. Anordnung nach Anspruch 2, **dadurch gekennzeichnet, dass** die Einführöffnung (3) eine ellipsenähnliche Form aufweist und in einer gedachten gewölbten Fläche (F) liegt, welche schräg zum hohlzylinderähnlichen Penisschaft (1a) des Kunstpenis (1) verläuft,
und dass die Durchsteck-Öffnung (7) in der Wandung des unteren Bereiches (1 ab) des Schaftes (1 a) des Kunstpenis (1) angeordnet ist, auf den die Einführöffnung (3) projizierbar ist.

4. Anordnung nach Anspruch 1, **dadurch gekennzeichnet, dass** ein vom vorderen Teil des eichelförmigähnlichen Aufnahmeraums (2a) ausgehender aus dem vorderen Teil des Kunstpenis (1) austretender Sperma-Kanal (5) vorgesehen ist.

5. Anordnung nach Anspruch 1, **dadurch gekennzeichnet, dass** eine Entlüftungsrinne (6) vorgehen ist, welche an der Innenwandung des Aufnahmeraumes (2a) und des Aufnahmeraumes (2b) bis zur Einführöffnung (3) verläuft.

6. Anordnung nach Anspruch 1, **dadurch gekennzeichnet, dass** das elastische Material des Kunstpenis (1) Silikon ist.

7. Anordnung nach Anspruch 1, **dadurch gekennzeichnet, dass** eine Entlüftungsrinne (6') vorgesehen ist, welche an der Innenwandung des Aufnahmeraumes für die Eichel des erigierten natürlichen Penis und des Aufnahmeraumes (2b') für den Penisschaft des erigierten natürlichen Penis verläuft und durch mindestens eine Entlüftungsöffnung (8') in der Wandung (1w') des Kunstpenis (1') mit dem Bereich außerhalb des Kunstpenis verbunden ist.
